# EUROPEAN PATENT SPECIFICATION

(11) **EP 2 822 615 B1**
(45) Date of publication and mention of the grant of the patent: **10.06.2020**
(21) Application number: 13757634.4
(22) Date of filing: 07.03.2013
(51) Int. Cl.: A61M 5/158, A61M 5/32

(54) **HUBER NEEDLE ASSEMBLY**
HUBERKANÜLENANORDNUNG
ENSEMBLE D'AIGUILLE DE HUBER

(30) Priority: 07.03.2012 US 201261607845 P; 03.10.2012 US 201261709286 P
(43) Date of publication of application: 14.01.2015
(73) Proprietor: Medical Components, Inc., Harleysville, PA 19438 (US)
(72) Inventor: FISHER, Mark, Harleysville, PA 19438 (US); LINDEN, Christopher, Harleysville, PA 19438 (US); CIUCIU, Cristian, M., Harleysville, PA 19438 (US); BIZUP, Raymond, R., Harleysville, PA 19438 (US)
(74) Representative: Molnia, David
(86) International application number: PCT/US2013/029625
(87) International publication number: WO 2013/134508

(56) References cited:
- EP-A1- 2 827 924
- WO-A1-2010/101573
- WO-A1-2010/101573
- US-A- 4 978 344
- US-A- 5 059 180
- US-A- 5 611 781
- US-A- 5 611 781
- US-A1- 2008 119 795
- US-A1- 2008 119 795
- US-A1- 2008 171 986
- US-A1- 2009 163 875

## Description

### CROSS REFERENCE TO RELATED APPLICATIONS

This application claims the benefit of U.S. Provisional Application No. 61/607,845, entitled "Huber Needle Assembly" and filed March 7, 2012, and U.S. Provisional Application No. 61/709,286, entitled "Huber Needle Assembly" and filed October 3, 2012.

### FIELD OF INVENTION

The present invention relates generally to needles for subcutaneous injections and, more specifically, to a Huber needle assembly comprising a tip block mechanism and a safety or restraint mechanism.

### BACKGROUND OF THE INVENTION

Conventional Huber needle assemblies are widely used in hospitals and alternate care sites and are often used in conjunction with implanted ports. Such Huber needle assemblies provide non-coring Huber needles that are used to administer chemotherapy, IV fluids, medications, total parenteral nutrition, or to transfuse blood products through the implanted ports. The implanted port contains a self-sealing septum that seals around the non-coring needle, holds the needle in place, and allows for multiple accesses by a Huber needle.

Conventional Huber needle assemblies are designed for safety of patients. They do, however, present considerable risks to the medical practitioners using them. A conventional Huber needle assembly requires two hands to extract its Huber needle from an implanted port. One hand is used to stabilize the implanted port, while the other hand is used to withdraw the needle. The force required to withdraw the needle from the self-sealing septum of the implanted port can cause the needle to rebound and stick the user. This may result in transfer of a blood-borne pathogen to the user. Further, it may expose the user to hazardous drugs.

Although several alternate Huber needle assemblies are available, a need still exists for a Huber needle assembly with safety features that minimize the risk of exposure to blood-borne pathogens or drugs.

### SUMMARY OF THE INVENTION

The invention is defined by the subject-matter of claim 1, wherein particular embodiments are defined in the dependent claims. In accordance with an aspect of the invention, there is provided a Huber needle assembly, which comprises a housing, a Huber needle disposed in the housing, and a base through which the Huber needle is slidably disposed. The Huber needle comprises a tip. The base comprises a needle tip blocker slidably disposed within the base to block the tip of the Huber needle when the tip of the Huber needle is retracted into the base.

In accordance with another aspect of the invention, there is provided a Huber needle assembly, which comprises a housing, a Huber needle, a base through which the Huber needle is slidably disposed, and a cable comprising a first end anchored within the housing and a second end anchored within the base. The base comprises a tip block mechanism configured to block the tip of the Huber needle when the tip of the Huber needle is retracted into the base. The cable is configured to restrain movement of the housing relative to the base.

In accordance with another aspect of the invention, there is provided a Huber needle assembly, which comprises a housing, a Huber needle, a base through which the Huber needle is slidably disposed, and a restraint means for restraining movement of the housing relative to the base. The base comprises a needle tip block means for blocking the tip of the Huber needle when the tip of the Huber needle is retracted into the base.

WO 2010/101573 A1 discloses a needle-based medical device and related method.

US 5,059,180 A discloses an automatic needle tip guard.

### BRIEF DESCRIPTION OF THE DRAWINGS

Reference to the drawings illustrating various views of exemplary embodiments of the present invention is now made. In the drawings and the description of the drawings herein, certain terminology is used for convenience only and is not to be taken as limiting the embodiments of the present invention. The terms, "distal" and "proximal," refer, respectively, to directions away from and closer to the surgeon inserting the insertion device described herein into a patient. Furthermore, in the drawings and the description below, like numerals indicate like elements throughout.
FIG. 1 illustrates a perspective view of an exemplary embodiment of a Huber needle assembly comprising a base, a housing for a Huber needle, a needle tip block mechanism, and a needle safety or restraint mechanism, in accordance with an exemplary embodiment not part of the present invention;
FIG. 2 illustrates a top perspective view of the base of the Huber needle assembly of FIG. 1, in accordance with an exemplary embodiment not part of the present invention;
FIG. 3 illustrates a bottom perspective view of the housing for the Huber needle of the Huber needle assembly of FIG. 1, in accordance with an exemplary embodiment not part of the present invention;
FIG. 4A illustrates a view of the Huber needle assembly of FIG. 1 from a cross-section A-A illustrated in FIG. 1, the cross-section A-A showing a spring of the needle tip block mechanism, in accordance with an exemplary embodiment not part of the present invention;
FIG. 4B illustrates a view of the Huber needle assembly of FIG. 1 from a cross-section B-B illustrated in FIG. 1, the cross-section B-B showing portions of the needle tip block mechanism and the needle safety or restraint mechanism, in accordance with an exemplary embodiment not part of the present invention;
FIG. 4C illustrates a view of the Huber needle assembly of FIG. 1 from a cross-section C-C illustrated in FIG. 1, the cross-section C-C showing portions of the needle safety or restraint mechanism, in accordance with an exemplary embodiment not part of the present invention;
FIG. 4D illustrates a perspective skeleton view of the Huber needle assembly of FIG. 1 showing portions of the needle safety or restraint mechanism, in accordance with an exemplary embodiment not part of the present invention.
FIG. 5 illustrates an exemplary embodiment of the needle tip block mechanism and the needle safety or restraint mechanism from the cross-section B-B of the Huber needle assembly of FIG. 1, the needle tip block mechanism comprising a ball and spring in a first position in which the Huber needle is capable of sliding through a bottom orifice of the base, in accordance with an exemplary embodiment not part of the present invention;
FIG. 6 illustrates a close-up view of the exemplary embodiment of the needle tip block mechanism of FIG. 5 in the base of the Huber needle assembly of FIG. 1 in a second position in which the Huber needle is prevented from exiting the base through the bottom orifice, in accordance with an exemplary embodiment not part of the present invention;
FIG. 7 illustrates a view of the Huber needle assembly of FIG. 1 from the cross-section B-B illustrated in FIG. 1, the cross-section B-B showing the exemplary embodiment of the needle tip block mechanism of FIG. 5 in the second position in which the Huber needle is prevented from exiting the base through the bottom orifice, in accordance with an exemplary embodiment not part of the present invention;
FIG. 8 illustrates a view of the Huber needle assembly of FIG. 1 as it is being inserted into an infusion port implanted within a patient, the infusion port and patient shown in cross-section, in accordance with an exemplary embodiment not part of the present invention;
FIG. 9 illustrates a view of the Huber needle assembly of FIG. 1 inserted into the infusion port of FIG. 8, in accordance with an exemplary embodiment not part of the present invention;
FIG. 10 illustrates a view of the Huber needle assembly of FIG. 1 as it is being withdrawn from the infusion port of FIG. 8, in accordance with an exemplary embodiment not part of the present invention;
FIG. 11 illustrates a view of the Huber needle assembly of FIG. 1 fully withdrawn from the infusion port of FIG. 8, in accordance with an exemplary embodiment not part of the present invention;
FIG. 12 illustrates a perspective view of another exemplary embodiment of a Huber needle assembly comprising a base, a housing for a Huber needle, another embodiment of a needle tip block mechanism, and another embodiment of a needle safety or restraint mechanism, in accordance with an exemplary embodiment not part of the present invention;
FIG. 13A illustrates a top perspective view of the base of the Huber needle assembly of FIG. 12 showing the needle safety or restraint mechanism, in accordance with an exemplary embodiment not part of the present invention;
FIG. 13B illustrates a top perspective view of the base of the Huber needle assembly of FIG. 12 with the needle safety or restraint mechanism removed, in accordance with an exemplary embodiment not part of the present invention;
FIG. 14 illustrates a bottom perspective view of the housing of the Huber needle assembly of FIG. 12, in accordance with an exemplary embodiment not part of the present invention;
FIG. 15A illustrates a view of the Huber needle assembly of FIG. 12 from a cross-section D-D illustrated in FIG. 12, in accordance with an exemplary embodiment not part of the present invention;
FIG. 15B illustrates a view of the Huber needle assembly of FIG. 12 from a cross-section E-E illustrated in FIG. 12, the cross-section E-E showing the needle tip block mechanism and the needle safety or restraint mechanism, in accordance with an exemplary embodiment not part of the present invention;
FIG. 16 illustrates an exemplary embodiment of the needle tip block mechanism and the needle safety or restraint mechanism from the cross-section E-E of the Huber needle assembly of FIG. 12, the needle tip block mechanism comprising a ball and spring in a first position in which the Huber needle is capable of sliding through a bottom orifice of the base, in accordance with an exemplary embodiment not part of the present invention;
FIG. 17 illustrates a close-up view of the exemplary embodiment of the needle tip block mechanism in the base of the Huber needle assembly of FIG. 16 in a second position in which the Huber needle is prevented from exiting the base through the bottom orifice, in accordance with an exemplary embodiment not part of the present invention;
FIG. 18 illustrates a view of the Huber needle assembly of FIG. 12 from the cross-section E-E illustrated in FIG. 12, the cross-section E-E showing the exemplary embodiment of the needle tip block mechanism of FIG. 16 in the second position in which the Huber needle is prevented from exiting the base through the bottom orifice, in accordance with an exemplary embodiment not part of the present invention;
FIG. 19 illustrates a perspective view of yet another exemplary embodiment of a Huber needle assembly comprising a base, a housing for a Huber needle, yet another embodiment of a needle tip block mechanism, and yet another embodiment of a needle safety or restraint mechanism, in accordance with an exemplary embodiment not part of the present invention;
FIG. 20 illustrates a top perspective view of the base of the Huber needle assembly of FIG. 19, in accordance with an exemplary embodiment not part of the present invention;
FIG. 21 illustrates a bottom perspective view of the housing of the Huber needle assembly of FIG. 19, in accordance with an exemplary embodiment of the present invention;
FIG. 22A illustrates a view of the Huber needle assembly of FIG. 19 from a cross-section F-F illustrated in FIG. 19, in accordance with an exemplary embodiment of the present invention;
FIG. 22B illustrates a view of the Huber needle assembly of FIG. 19 from a cross-section G-G illustrated in FIG. 19, the cross-section G-G showing the needle tip block mechanism and the needle safety or restraint mechanism, in accordance with an exemplary embodiment of the present invention;
FIG. 23 illustrates an exemplary embodiment of the needle tip block mechanism and the needle safety or restraint mechanism from the cross-section G-G of the Huber needle assembly of FIG. 19, the needle tip block mechanism comprising a spring detent in a first position in which the Huber needle is capable of sliding through a bottom orifice of the base, in accordance with an exemplary embodiment of the present invention;
FIG. 24 illustrates a close-up view of the exemplary embodiment of the needle tip block mechanism in the base of the Huber needle assembly of FIG. 24 in a second position in which the Huber needle is prevented from exiting the base through the bottom orifice, in accordance with an exemplary embodiment of the present invention;
FIG. 25 illustrates a view of the Huber needle assembly of FIG. 19 from the cross-section G-G illustrated in FIG. 19, the cross-section G-G showing the needle tip block mechanism of FIG. 24 in the second position in which the Huber needle is prevented from exiting the base through the bottom orifice, in accordance with an exemplary embodiment of the present invention;
FIG. 26 illustrates an exemplary alternative embodiment of the Huber needle assemblies of FIGS. 1, 12, and 19, in which the housing comprises a plurality of holes and the base comprises a plurality of detents, in accordance with an exemplary embodiment of the present invention; and
FIGS. 27A-C illustrate the housing and the base of the Huber needle assembly of FIG. 26 in various positions, in accordance with an exemplary embodiment of the present invention.

### DETAILED DESCRIPTION OF THE INVENTION

A Huber needle assembly desirably includes a needle safety or restraint mechanism and a needle tip block mechanism which together safely retain portions of the Huber needle that may contain blood products or medication within a needle impenetrable enclosure to reduce or minimize needle sticks to a medical practitioner.

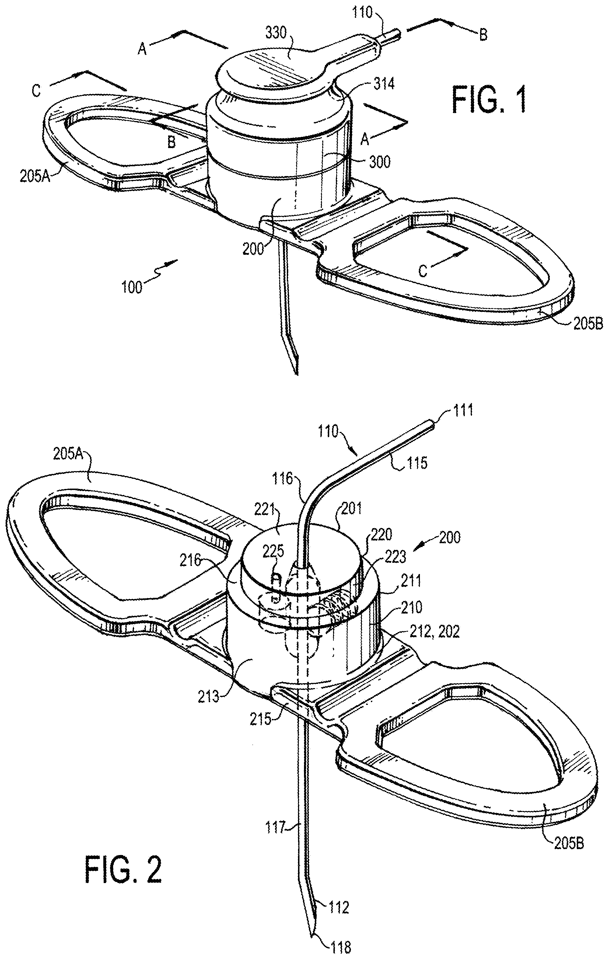

Referring now to FIG. 1, there is illustrated an exemplary embodiment of a Huber needle assembly, generally designated as 100 The Huber needle assembly 100 comprises a Huber needle 110, a base 200, and a housing 300 for the Huber needle 110. The Huber needle 110 is securely mounted within the housing 300 and is slidably disposed through the base 200. Except as otherwise described below, the components of the Huber needle assembly 100 are formed from plastic. The Huber needle 110 is formed from metal, such as stainless steel.

Illustrated in FIG. 2 is a perspective view of the base 200 and the Huber needle 110 slidably disposed therethrough. In FIG. 2, the housing 300 of the Huber needle assembly 100 has been removed to more clearly show the base 200. The base 200 has a first side or end 201 and a second side or end 202. The base 200 comprises a base plate 215 disposed at the second side 202 of the base 200, a pair of wings 205A and 205B connected to one another by the base plate 215, and a body 210. The body 210 comprises a first side or end 211, a second side or end 212, and an outer wall 213. The second end 212 of the body 210 is connected to the base plate 215. In an exemplary embodiment, the wings 205, the base body 210, and the base plate 215 form a unitary structure. It is to be understood that other embodiments in which such components are separate but connected either temporarily or permanently together are contemplated.

Disposed at the first side 201 of the base 200 is a protrusion 220 connected to the first side 211 of the body 210. The protrusion 220 comprises a side surface 223 and a top surface 221 having a hole 225. In an exemplary embodiment, the protrusion 220 forms a unitary structure with the base body 215, although alternative embodiments in which these components are separate are contemplated. The portion of the first side 211 of the base body 210 not covered by the protrusion 220 is a surface 216. The surface 216 has a ring or annular shape.

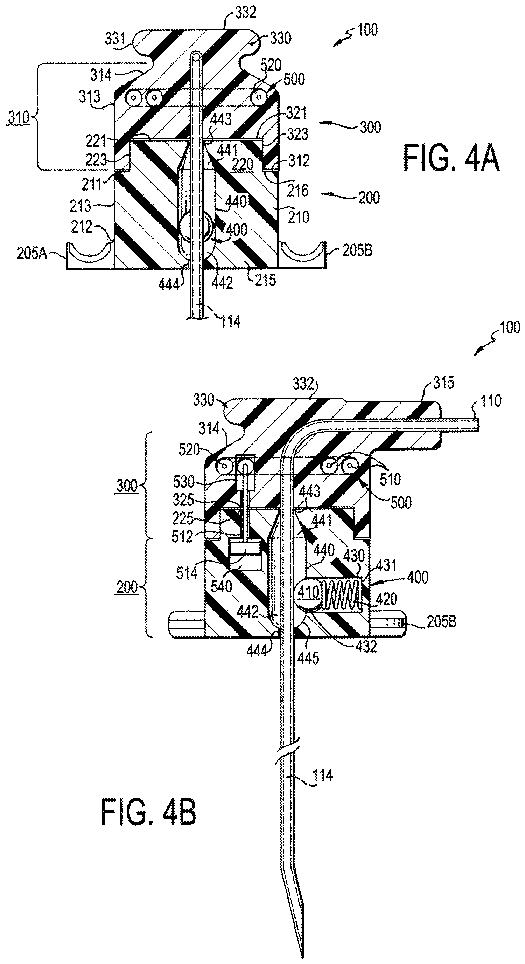

The Huber needle 110 is slidably disposed through the base 200. As illustrated in FIG. 2, the Huber needle 110 has a first end 111 and a second end 112. The Huber needle 110 comprises a first portion 115 at the first end 111, a second portion 117 at the second end 112, and a bend 116 between the first and second portions 115, 116. The Huber needle 110 includes an internal needle cannula 114 (illustrated in FIGS. 4A and 4B) that terminates at a sharp tip 118 at the second end 112. As described in further detail below, the Huber needle 110 is slidably disposed through the base 200.

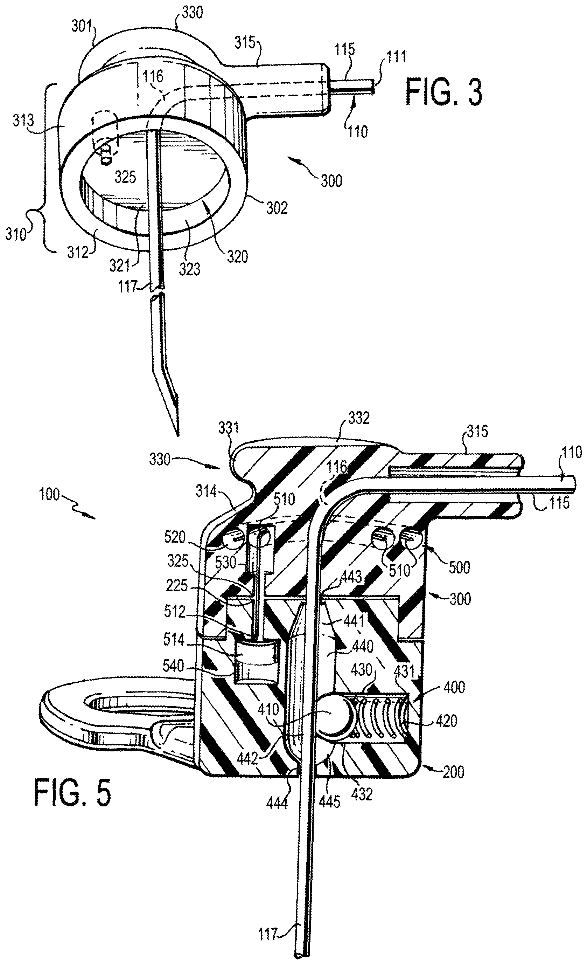

Illustrated in FIG. 3 is a perspective view of the housing or hub 300 for the Huber needle 110 from a bottom of the housing 300. The housing 300 is now described with reference to FIGS. 1 and 3.

The housing 300 has a first side or end 301 and a second side or end 302. The housing 300 comprises a body 310, a handle 330, and an arm 315. The handle 330 is disposed in the housing 300 at the first side 301 of the housing 300. The handle 330 is smoothly connected to the housing body 310 by a contoured surface 314. The handle 330 includes a flare 331 (labeled in FIG. 5) that assists a nurse or medical practitioner in using the Huber needle assembly 100 to insert and withdraw the tip 118 of the Huber needle 110 from a subcutaneously implanted venous access port. The handle 330 further includes a top surface 332 (illustrated in FIG. 5) upon which the nurse or medical practitioner may place his or her hand or digit when using the Huber needle assembly 100.

The arm 315 is connected to the handle 330. The first portion 115 of the Huber needle 110 is disposed through the arm 315. The arm 315 thereby provides support to the first portion 115 of the Huber needle 110. The first end 111 of the Huber needle 110 is connected to a catheter (not shown). The arm 315 also assists a nurse or medical practitioner in using the Huber needle assembly 100 to insert and withdraw the tip 118 of the Huber needle 110 from a subcutaneously implanted venous access port.

The body 310 of the housing 300 comprises an outer wall 313 and a bottom surface 312. Disposed within the body 310 at the second side 302 of the housing 300 is an interior space 320. The interior space 320 comprises an upper surface 321 and an inner wall 323. Disposed within the upper surface 321 is a hole 325, which is described in more detail below. Because the interior space 320 is disposed within the body 310 at the second side 302 of the housing 300, the bottom surface 312 of the body 310 has a ring or annular shape.

As seen in FIG. 3, the Huber needle 110 is securely mounted within the housing 300. The Huber needle 110 enters the housing 300 via the arm 315, bends within the housing, and exits the housing 300 at the upper surface 321 of the interior space 320. The bend 116 is fully encapsulated within the housing 300 and assists in anchoring the Huber needle 110 within the housing 300.

In the exemplary embodiment illustrated in FIGS. 1-3, the body 210 of the base 200 and the body 310 of the housing 300 each have a generally cylindrical shape. Thus, the outer wall 213 of the base body 210 and the outer wall 313 of the housing body 310 are each generally cylindrical. The side surface 223 of the protrusion 220 of the base 200 and the inner wall 323 of the interior space 320 of the housing 300 are also generally cylindrical. It is to be understood that other shapes and configurations for the bodies 210 and 310 are contemplated and that they are not limited to having cylindrical outer walls 213 and 313, respectively. Other shapes and configurations for the side surface 223 and the inner wall 323 are also contemplated. Such other shapes include rectangular prism, rounded rectangular prism, etc.

FIG. 4A illustrates a cross-sectional view of the Huber needle assembly 100 taken along a line A-A illustrated in FIG. 1. FIG. 4B illustrates a cross-sectional view of the Huber needle assembly 100 taken along a line B-B illustrated in FIG. 1. FIGS. 4A and 4B illustrate how the base 200 mates with the housing 300. FIGS. 4A and 4B are now described together with reference to FIG. 3.

The protrusion 220 of the base 200 is sized to fit within the interior space 320 of the housing 300. When so situated, the top surface 221 of the protrusion 220 is adjacent to the upper surface 321 of the interior space 320, and the rim 216 is adjacent to the bottom surface 312 of the body 310. Additionally, the interior wall 323 of the interior space 320 is adjacent to the side surface is 223 of the protrusion 220. In the exemplary embodiment illustrated in FIGS. 4A and 4B, there a small gap between the side surface 223 and the inner wall 323 and a gap between the top surface 221 and the upper surface 321. A detent on the side surface 223 may be used to engage a corresponding dent on the inner wall 323 to maintain the gaps during use. In an alternative exemplary embodiment, no such gaps exist such that when the base 200 is mated to the housing 300, the side surface 223 is in contact with the inner wall 323, and the top surface 221 is in contact with the upper surface 321. In such exemplary embodiment, the base 200 may fit tightly to the housing 300 but be separable from the housing 300 during use.

The Huber needle assembly 100 includes a needle capture mechanism comprising a tip block mechanism 400 and a safety or restraint mechanism 500. An exemplary embodiment of the tip block mechanism 400 is illustrated in FIGS. 4A and 4B. An exemplary embodiment of the safety or restraint mechanism 500 is illustrated in FIGS. 4A-4D.

Referring to FIGS. 4A and 4B, the tip block mechanism 400 comprises a needle tip blocker 410 and a spring 420 which is configured to urge the needle tip blocker 410 to block the tip 118 of the Huber needle 110 when the Huber needle 110 is withdrawn from a patient. The tip block mechanism 400 further comprises a horizontal race (or chamber) 430 for the needle tip blocker 410 and a vertical needle chamber 440 through which the Huber needle 110 is disposed in the base 200. As described above, the Huber needle 110 is slidably disposed through the base 200.

In an exemplary embodiment, the needle tip blocker 410 is a metal, e.g., a variety of steel, ball and the spring 420 is a metal, e.g., a variety of steel, spring. In such embodiment, the horizontal race 430 is a horizontal ball race in which the ball 410 is slidably disposed or in which the ball 410 is rolled. It is to be understood that, in other exemplary embodiments, the needle block 410 may be a metal disc, flat metal plate or cup, a flexible metal shield, a hard plastic ball, etc. For purposes of discussion below, the needle tip blocker 410 is described as being a ball 410. It is to be understood that the needle tip blocker 410 is not so limited.

The Huber needle 110 is slidably disposed through the vertical needle chamber 440. The vertical needle chamber 440 comprises a first end 441 having an orifice 443 and a second end 442 having an orifice 444. The orifices 443 and 444 are sized to snuggly yet slideably fit the Huber needle 110 so the second portion 117 of the Huber needle 110 may slide through the orifices 443 and 444 and the vertical needle chamber 440. In an exemplary embodiment, the orifice 443 is sized so that a fluid seal is created between the Huber needle 110 and the orifice 443. The second end 442 of the vertical needle chamber 440 further comprises a cup 445 sized to accommodate the ball 410.

The horizontal ball race 430 is disposed within the base 200 perpendicularly to the vertical needle chamber 440. The horizontal ball race 430 comprises a first end 431 and a second end 432. The first end 431 is a dead end and is internal to the base 200. The second end 432 opens to the second end 442 of the vertical needle chamber 440. The spring 420 is disposed at the first end 431 of the horizontal ball race 430, and the ball 410 is disposed at the second end 432 of the horizontal ball race 430. The spring 420 urges the ball 410 toward the vertical needle chamber 440 and the Huber needle 110. When the Huber needle 110 is fully disposed through the vertical needle chamber 440, the spring 420 presses the ball 410 against the Huber needle 110. The Huber needle 110 blocks the ball 410 from fully entering the vertical needle chamber 440.

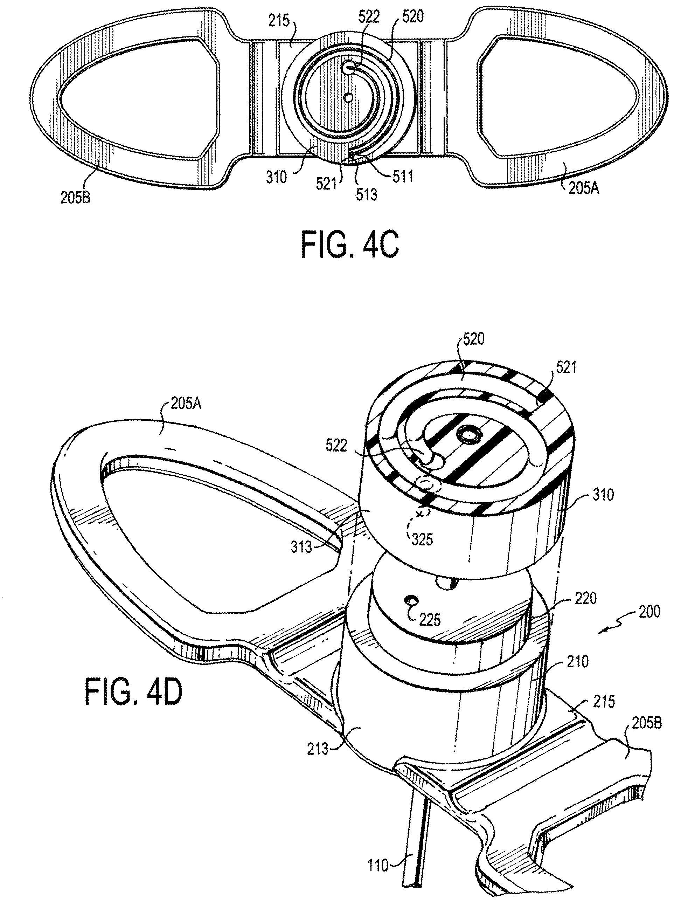

FIG. 4C illustrates a cross-sectional view of the Huber needle assembly 100 taken along a line C-C illustrated in FIG. 1. FIG. 4D illustrates a perspective skeleton view of the Huber needle assembly 100, in accordance with an exemplary embodiment of the present invention. Referring to FIGS. 4A-D, the safety or restraint mechanism 500 comprises cable 510 coupling the base 200 to the housing 300. The safety or restraint mechanism 500 limits movement of the base 200 relative to the housing 300.

The cable 510, seen in FIGS. 4B-4C, comprises a first end 511 having a first stop 513 and a second end 512 comprising a second stop 514. The stop 513 anchors the first end 511 of the cable 510 to the housing 300. The stop 514 anchors the second end 512 of the cable 510 to the base 200. The cable 510 is not illustrated in FIG. 4D for clarity of view of other aspects of the safety or restraint mechanism 500. The cable 510 may be formed from metal, e.g., a variety of steel, or suture material. It may be solid or stranded. Thus, the cable 510 may be a metal wire or a suture line. The stops 513 and 514 may be formed from the cable 510 or may be separate components permanently attached to the cable. In an exemplary embodiment, the stops 513 and 514 are formed from metal, e.g., a variety of steel. In another exemplary embodiment, they are formed from plastic.

The housing 300 comprises a coiled chamber 520 (best seen in FIGS. 4C and 4D) in which a portion of the cable 510 is disposed. The coiled chamber 520 comprises a first end 521 and a second end 522. The second end 522 opens to a vertical chamber 530 disposed within the housing 300. The vertical chamber 530 communicates to the hole 325 in the upper surface 321 of the interior space 320. The first end 521 dead ends in the housing 300.

Disposed within the base 200 is a vertical chamber 540 which communicates via the hole 225 to the top surface 321 of the protrusion 320. The second stop 514 is disposed within the vertical chamber 540 so that the second end 512 of the cable 510 is anchored within the base 200. The cable 510 ascends from the vertical chamber 540, through the hole 225, out of the base 200, and into the hole 325.

When the Huber needle assembly 100 is in its pre-insertion or insertion state in which the base 200 is adjacent to the housing 300, the cable 510 ascends through the hole 325 and through the vertical chamber 530. The cable 510 exits the top of the vertical chamber 530 and coils through the coiled chamber 520. The first stop 513 is disposed at the first end 521 of the coiled chamber 520.

When the Huber needle assembly 100 is in its withdrawal or post-insertion state in which the base 200 is separated from the housing 300, the first end 511 of the cable 510 has been pulled through the coiled chamber 520 from the first end 521 to the second end 522. Movement of the first end 511 is ended when the first stop 513 reaches the vertical chamber 530.

The positions of the components of the tip block mechanism 400 and the safety or restraint mechanism 500 in the pre-insertion and post-insertion states of the Huber needle assembly 100 are now described with respect to FIGS. 5 though 7. Referring now to FIG. 5, there is illustrated a perspective view of the Huber needle assembly 100 from the cross-section B-B of FIG. 1, in accordance with an exemplary embodiment of the present invention. FIG. 5 illustrates the Huber needle assembly 100 in its pre-insertion or insertion state in which the base 200 is adjacent to the housing 300. In this state, the ball 410 is in place within the horizontal ball race 430 at the second end 432 thereof. The ball 410 is in contact with the second portion 117 of the Huber needle 110. The spring 420 provides a lateral force to press the ball 410 against the Huber needle 110.

In this state, the cable 510 is coiled through the coiled chamber so that the first stop 513 is disposed at the first end 521 of the coiled chamber 520. As can be seen in FIG. 5, the second stop 514 of the cable 510 is disposed within the vertical chamber 540. The diameter of the stop 514 is wider than the hole 225 so that the stop 514 cannot be pulled out from the vertical chamber 540. The second end 512 of the cable 510 is thereby limited from leaving the base 200. Because the cable 510 is coiled through coiled chamber 520 in the housing 300, the housing 300 may be separated from the base 200. The first end 511 of the cable 510 is slidably disposed in the coiled chamber 520, and the ball 410 is not blocking the orifice 444.

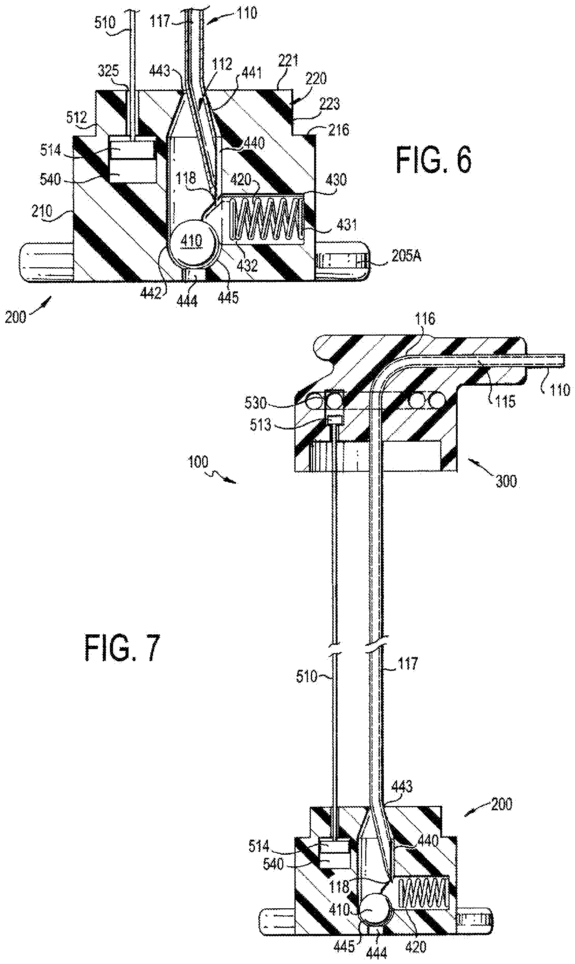

FIGS. 6 and 7 illustrate a perspective view of the base 200 and the Huber needle assembly 100, respectively, from the cross-section B-B of FIG. 1. FIG. 6 shows a close-up view of the cross-section of the base 200 of the Huber needle assembly 100 in its post-insertion position in which the housing 300 has been pulled away from the base 200, and FIG. 7 illustrates a view of the cross-section of the entire Huber needle assembly 100 in its post-insertion position in which the housing 300 has been pulled away from the base 200.

As seen in FIGS. 6 and 7, the tip 118 of the Huber needle 110 is contained within the vertical needle chamber 440 when the housing 300 has been fully pulled away from the base 200. In such position, the spring 420 has pressed the ball 410 into the cup 445 of the vertical needle chamber 440, thereby blocking the orifice 444 at the second end 442 of the vertical needle chamber 440. The tip 118 of the Huber needle 110 is thereby blocked from passing through the orifice 444. If the Huber needle 110 is pressed toward the orifice 444, the ball 410 blocks the tip 118 of the Huber needle 110 or deflects it into the cup 445 or the wall of the vertical needle chamber 440. In this way, the tip block mechanism 400 minimizes the risk of accidental needle sticks by blocking the tip 118 from exiting the orifice 444.

When the housing 300 has been pulled away from the base 200 so that the tip 118 of the Huber needle 110 is restrained within the vertical needle chamber 440, the first stop 513 at the first end 511 of the cable 510 is captured within the vertical chamber 530, and the second end 512 of the cable 510 remains within the vertical chamber 540. The cable 510 spans between the base 200 and the housing 300. Further lateral movement of the base 200 away from the housing 300 is restrained by the fact that the stops 513 and 514 are captured within their respective vertical chambers 530 and 540. Thus, the safety or restraint mechanism 500 prevents further lateral movement of the base 200 away from the housing 300 such that the Huber needle 110 tip 118 may not be pulled up out of the orifice 443 of the vertical needle chamber 440. In this way, the safety or restraint mechanism 500 minimizes the risk of accidental needle sticks by restraining movement of the Huber needle 110 relative to the base 300.

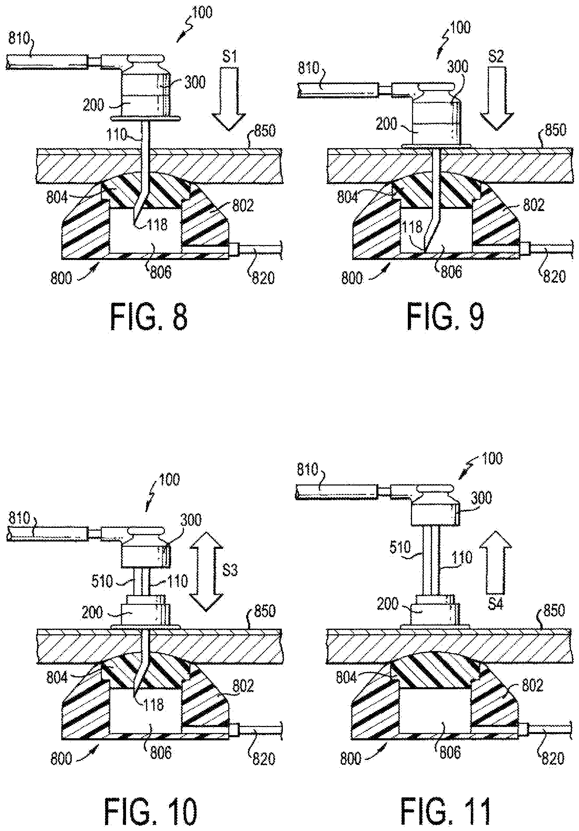

FIGS. 8-11 illustrate exemplary steps S1-S4 for inserting and withdrawing the Huber needle assembly 100 from a subcutaneous infusion port 800 implanted within a patient. The subcutaneous infusion port 800, which is illustrated in cross-section in the figures, comprises a body 802 having a reservoir 806 fluidly sealed by a needle penetrable septum 804. In an exemplary embodiment, the needle penetrable septum 804 is formed from silicone, and the body 802 is formed from polysulfone. For purposes of illustration, the skin and tissue 850 of the patient is also shown in cross-section in the figures.

Referring now to FIG. 8, there is illustrated a Step S1 in which the Huber needle assembly 100 is being inserted through the skin and tissue 850 of a patient and into the subcutaneous infusion port 800. The Huber needle 110 is pressed through the skin and tissue 850 and the septum 804 of the subcutaneous infusion port 800, and the tip 118 of the Huber needle 110 enters the reservoir 806 of the infusion port 800. As shown in the figure, the housing 300 is in contact with the base 200 of the Huber needle assembly 100 when the Huber needle assembly 100 is in its insertion state.

Referring to FIG. 9, there is illustrated a Step S2 in which the Huber needle assembly 100 is fully inserted through the skin and tissue 850 and the septum 804. The base 200 of the Huber needle assembly 100 is shown resting against the skin 850, and the tip 118 of the Huber needle assembly 100 is fully inserted into the reservoir 806 of the subcutaneous infusion port 800. The Huber needle assembly 100 is in a proper position for infusing fluid into the port 800. The first end 111 (labeled in FIG. 2) of the Huber needle 110 is connected to a catheter tube 810. The Huber needle assembly 100 may then infuse fluids from the catheter 810 into the reservoir 806 of the subcutaneous infusion port 800. The fluids are delivered to a portion of patient's body via a catheter 820 connected to the port 800.

Referring to FIG. 10, after infusion is complete, Huber needle 110 is withdrawn from the port 800 in a Step S3. The medical practitioner using the Huber needle assembly 100 holds the wings 205A and 205B of the Huber needle assembly 100 against the skin 850 while pulling the housing 300 via the handle 330 (and, optionally, the arm 315) away from the patient. The medical practitioner holds down the wings 205A and 205B of the base 200 with one hand so that the base 200 remains substantially against the skin 850 while the Huber needle 110 is withdrawn. With the other hand, the medical practitioner pulls the housing 300 of the Huber needle assembly 100 away from the base 200 and the infusion port 800. Because the medical practitioner holds the base 200 against the skin 850 with one hand and pulls the housing 300 away from the skin 850 with the other hand, the housing 300 lifts away from the base 200.

As a gap develops between the housing 300 and the base 200, the first stop 513 at the first end 511 of the cable 510 is pulled from the first end 521 of the coiled chamber 520. As the medical practitioner continues to pull the housing 300 away from the base 200, the first end 511 of the cable 510 is pulled through the coiled chamber 520 toward the second end 522 of the coiled chamber 520. The cable 510 can be seen in FIG. 10 between the housing 300 and the base 200.

Referring now to FIG. 11, there is illustrated the Huber needle 110 of the Huber needle assembly 100 fully withdrawn from the port 800 in a Step S4. The tip 118 of the Huber needle 110 is disposed within the base 200 of the Huber needle assembly 100, and the cable 510 is extended between the housing 300 and the base 200 of the Huber needle assembly 100. The tip 118 of the Huber needle 110 is blocked by the ball 410 from passing through the orifice 444 in the base 200. The cable 510 prevents further lateral movement of the housing 300 away from the base 200 so that the Huber needle 110 tip 118 may not be pulled up out of the orifice 443 of the vertical needle chamber 440. Thus, the tip 118 of the Huber needle 110 is captured within the base 200, and the Huber needle assembly 100 thereby minimizes the risk of accidental needle stick to the medical practitioner and the patient.

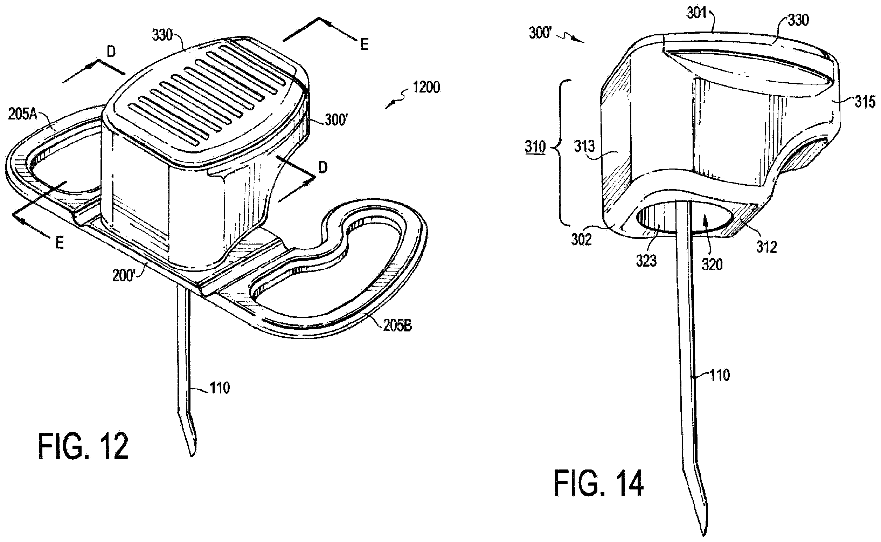

Illustrated in FIG. 12 is an alternative exemplary embodiment of the Huber needle assembly 100, generally designated as 1200 in the figure. The Huber needle assembly 1200 includes numerous elements in common with the Huber needle assembly 100. Like elements are labeled with the same numerals. It is to be understood that not all of such like elements are identical in form, shape, or function. Several of such elements of the Huber needle assembly 1200, though having similar forms, shapes, or functions to elements of the Huber needle assembly 100 having the same numerals, do not have identical forms, shapes, or functions. Several differences in such forms, shapes, or functions are described below. It is to be understood that the description below of such differences is not to be construed as a discussion of all differences between elements of the Huber needle assemblies 100 and 1200. Instead, differences may be apparent from the figures and are not addressed below.

The Huber needle assembly 1200 comprises the Huber needle 110, a base 200', and a housing 300' for the Huber needle 110. The Huber needle 110 is securely mounted within the housing 300' and is slidably disposed through the base 200'. Except as otherwise described below, the components of the Huber needle assembly 1200 are formed from plastic. The Huber needle 110 is formed from metal, such as stainless steel.

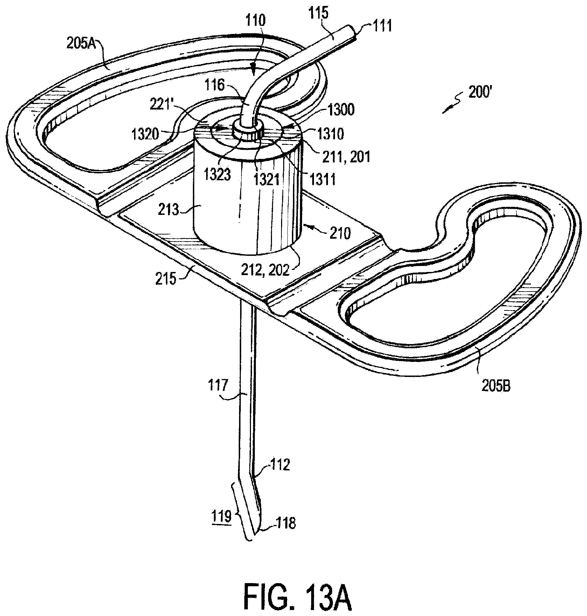

Illustrated in FIG. 13A is a perspective view of the base 200' and the Huber needle 110 slidably disposed therethrough. In FIG. 13A, the housing 300' of the Huber needle assembly 1200 has been removed to more clearly show the base 200'. The base 200' has a first side or end 201 and a second side or end 202. The base 200' comprises the base plate 215 disposed at the second side 202 of the base 200', the pair of wings 205A and 205B connected to one another by the base plate 215, and the body 210. The base 200' has numerous other similarities with the base 200. For example, the body 210 is generally cylindrical in shape; the Huber needle 110 is slidably disposed through the base 200'; etc.

The base 200' differs from the base 200 in a few ways. One difference between the bases 200 and 200' is that the base 200' includes a collar 1310, which is not present in exemplary embodiment of the base 200 illustrated in FIG. 2. As described in further detail below, the collar 1310 is part of a safety or restraint mechanism 1300, which restrains movement of the housing 300' away from the base 200' to prevent the tip 118 of the Huber needle 110, specifically a bent portion 119 at the tip 118 of the Huber needle 110, from being pulled from the base 200'. The safety or restraint mechanism 1300 performs the functions of the safety or restraint mechanism 500 of the Huber needle assembly 100, without use of the cable 510, the first stop 513, the second stop 514, or the holes 225 and 325 present in the Huber needle assembly 100. In an exemplary embodiment, the safety or restraint mechanism 1300 is formed from metal, e.g., a variety of steel.

Comparing FIG. 13A to FIG. 2, other differences are observable. For example, the wings 205A and 205B of the base 200' have a different shape from the wings 205A and 205B of the base 200. Further, the base 200' does not include the protrusion 220 present on the base 200. Rather, the collar 1310 comprises a protrusion 1320 having a cylindrical wall 1323 and a top surface 1321. The top surface 221' of the base 200' is an annular surface that is planar with the top surface 1311 of the collar 1310.

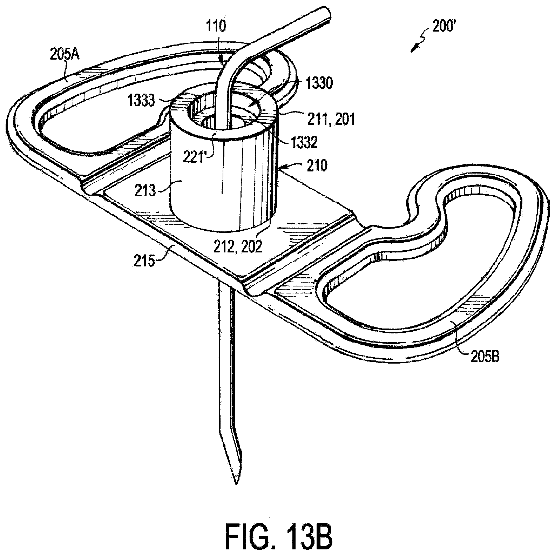

Referring now to FIG. 13B, there is illustrated a perspective view of the base 200' in which the collar 1310 is removed. FIG. 13B shows that the base 200' comprises a cylindrical space 1330 defined by an interior cylindrical wall 1333 and an annular bottom surface 1332. The cylindrical space 1330 is sized and shaped to accommodate the collar 1310. The annular bottom surface 1332 serves as a seat for the collar 1310.

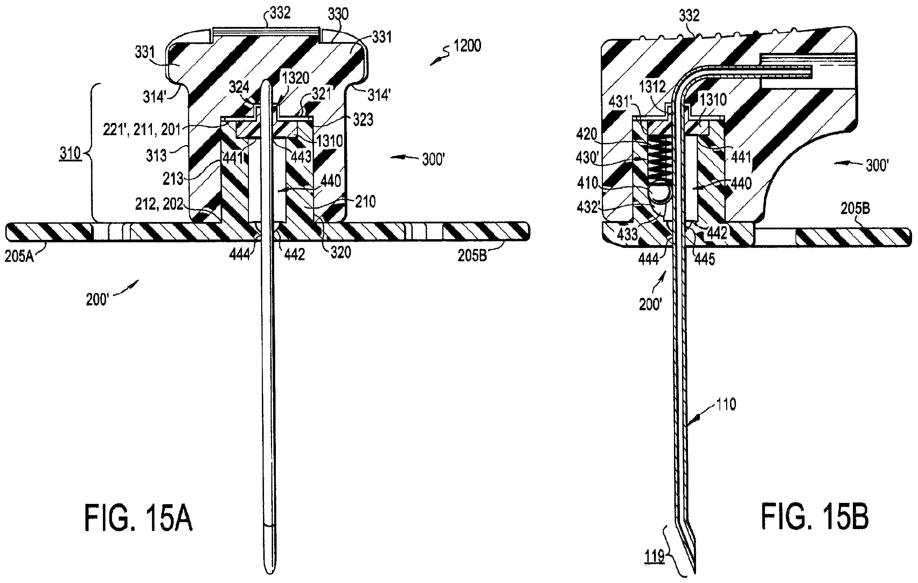

Illustrated in FIG. 14 is a perspective view of the housing or hub 300' for the Huber needle 110 from a bottom of the housing 300'. The housing 300' comprises the handle 330, which is shaped differently from the handle 330 of the housing 300. The handle 330 includes the top surface 332 (shown in FIGS. 15A and 15B) upon which the nurse or medical practitioner may place his or her hand or digit when using the Huber needle assembly 1200.

FIG. 15A illustrates a cross-sectional view of the Huber needle assembly 1200 taken along a line D-D illustrated in FIG. 12. FIG. 15B illustrates a cross-sectional view of the Huber needle assembly 1200 taken along a line E-E illustrated in FIG. 12. FIGS. 15A and 15B illustrate how the base 200' mates with the housing 300'. FIGS. 15A and 15B are now described together with reference to FIG. 13A.

The Huber needle 110 is securely mounted within the housing 300'. The Huber needle 110 enters the housing 300' via the arm 315, bends within the housing, and exits the housing 300' at the upper surface 321 of the interior space 320. The bend 116 is fully encapsulated within the housing 300 and assists in anchoring the Huber needle 110 within the housing 300'.

The body 210 of the base 200' has a generally cylindrical shape for fitting within the generally cylindrical interior space 320 of the housing 300'. The body 310 of the housing 300', however, does not have a generally cylindrical shape, unlike the body 310 of the housing 300. Instead, the outer wall 313 of the body 310 has a general rectangular prism shape. It is to be understood that other shapes and configurations for the bodies 210 and 310 of the base 200' and housing 300', respectively, are contemplated and that they are not limited to the shapes illustrated in the figures.

The protrusion 1320 of the collar 1310 is sized to fit within an interior recess 324 located on the top surface 321 of the interior space 320 of the housing 300'. When so situated, the top surface 1321 of the protrusion 1320 is adjacent to the upper surface of the interior recess 324, and the rim 221' is adjacent to the upper surface 321 of the interior space 320 of the housing 300'. Additionally, the interior wall 323 of the interior space 320 is adjacent to the outer wall 213. In the exemplary embodiment illustrated in FIGS. 15A and 15B, there a gap between the outer wall 213 and the inner wall 323 and a gap between the rim 221'/collar 1310 and the upper surface 321. A detent on the outer wall 213 may be used to engage a corresponding dent on the inner wall 323 to maintain the gaps during use. In an alternative exemplary embodiment, no such gaps exist such that when the base 200' is mated to the housing 300', the outer wall 213 is in contact with the inner wall 323, and the rim 221'/collar 1310 is in contact with the upper surface 321. In such exemplary embodiment, the base 200' may fit tightly to the housing 300' but be separable from the housing 300' during use.

The Huber needle assembly 1200 includes a needle capture mechanism comprising a tip block mechanism 400' and the safety or restraint mechanism 1300. An exemplary embodiment of the tip block mechanism 400' is illustrated in FIGS. 15A and 15B. Generally, the tip block mechanism 400' is similar to the tip block mechanism 400. A primary difference between the two is that the horizontal ball race 430 in the Huber needle assembly 100 is replaced by a vertical ball race (or chamber) 430' in the Huber needle assembly 1200 and that the orifice 443 at the first end 441 of the vertical needle chamber 440 is replace by an orifice or passageway 1312 through the collar 1310, which is disposed at the first end 441 of the vertical needle chamber 440 of the Huber needle assembly 1200.

The vertical ball race 430' is disposed within the base 200' in parallel with the vertical needle chamber 440. The vertical ball race 430' comprises a first end 431', which is capped by the needle limiter 1310 and a second end 432'. The first end 431' is a dead end and is internal to the base 200'. The second end 432' opens to the second end 442 of the vertical needle chamber 440. The spring 420 is disposed at the first end 431' of the vertical ball race 430', and the ball 410 is disposed at the second end 432 of the vertical ball race 430'. The spring 420 urges the ball 410 toward the vertical needle chamber 440 and the Huber needle 110 because the second end 432' of the vertical ball race 430' includes a ramp 433 angled toward the vertical needle chamber 440. When the Huber needle 110 is fully disposed through the vertical needle chamber 440, the spring 420 presses the ball 410 against the ramp 433, which in turn deflects the ball 410 into Huber needle 110. The Huber needle 110 blocks the ball 410 from fully entering the vertical needle chamber 440.

When the Huber needle assembly 1200 is in its pre-insertion or insertion state in which the base 200' is adjacent to the housing 300', the Huber needle 110 is slidably disposed through the hole 1312 in the collar 1310. When the Huber needle assembly 1200 is in its withdrawal or post-insertion state in which the base 200' is separated from the housing 300', the bend 119 of the Huber needle 110 is prevented from passing through the hole 1312 in the collar 1310. In an exemplary embodiment, the base 200' of the Huber needle assembly 1200 is sized so that the bend 119 of the Huber needle 110 binds up within the hole 1312, rather than merely being prevented from entering the hole 1312.

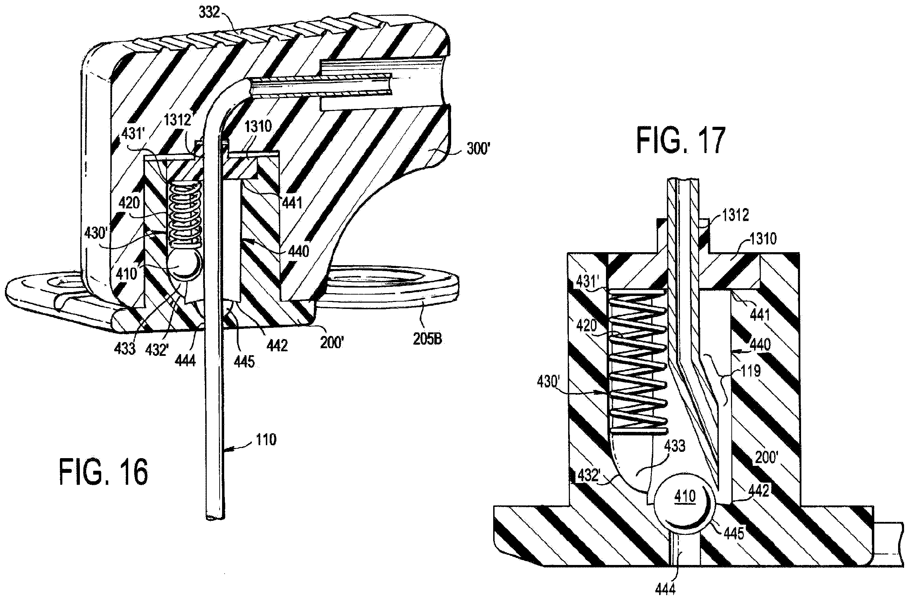

The positions of the components of the tip block mechanism 400' and the safety or restraint mechanism 1300 in the pre-insertion and post-insertion state of the Huber needle assembly 1200 are now described with respect to FIGS. 16 through 18. FIG. 16 illustrates a perspective view of the Huber needle assembly 1200 from the cross-section E-E of FIG. 12. Specifically, FIG. 16 illustrates the Huber needle assembly 1200 in its pre-insertion or insertion state in which the base 200' is adjacent to the housing 300'. In this state, the ball 410 is in place within the vertical ball race 430' at the second end 432' thereof. The ball 410 is in contact with the second portion 117 (labeled in FIG. 13A) of the Huber needle 110. The spring 420 provides a vertical force to press the ball 410 against the ramp 433 which presses the ball 410 against the Huber needle 110.

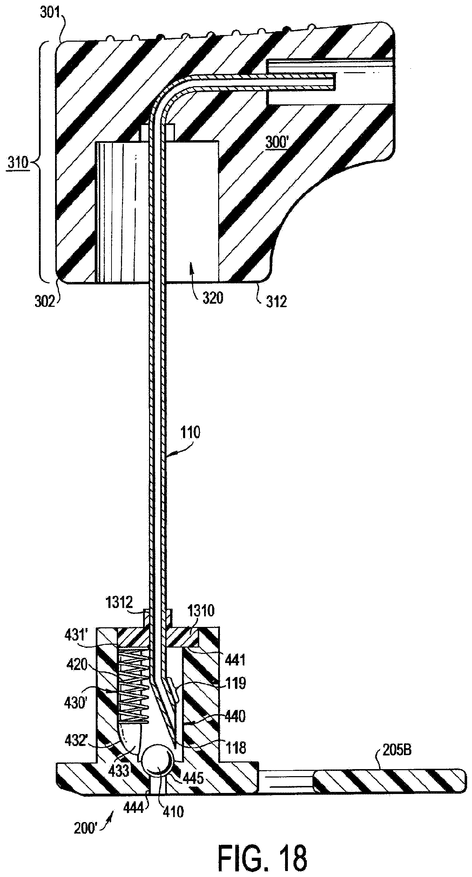

FIGS. 17 and 18 illustrate a perspective view of the base 200' and the Huber needle assembly 1200, respectively, from the cross-section E-E of FIG. 12. FIG. 17 illustrates the base 200' of the Huber needle assembly 1200 in its post-insertion position in which the housing 300' has been pulled away from the base 200', and FIG. 18 illustrates the Huber needle assembly 1200 in its post-insertion state in which the housing 300' has been pulled away from the base 200'.

As seen in FIGS. 17 and 18, the tip 118 of the Huber needle 110 resides within the vertical needle chamber 440 when the housing 300' has been fully pulled away from the base 200'. In such position, the spring 420 has pressed the ball 410 against the ramp 433 of the vertical ball race 430' and into the cup 445 of the vertical needle chamber 440, thereby blocking the orifice 444 at the second end 442 of the vertical needle chamber 440. The tip 118 of the Huber needle 110 is thereby blocked from passing through the orifice 444. In this way, the tip block mechanism 400' minimizes the risk of accidental needle sticks by blocking the tip 118 from exiting the orifice 444.

When the housing 300' has been pulled away from the base 200' so that the tip 118 of the Huber needle 110 is restrained within the vertical needle chamber 440, the bend 119 of the Huber needle 110 comes into contact with the collar 1310. The bend 119 of the Huber needle 110 is prevented from passing through the hole 1312 in the collar 1310 as it is larger than the diameter of the hole 1312. Further lateral movement of the base 200' away from the housing 300' is prevented by the fact that the bend 119 cannot pass through the hole 1312. In this way, the safety or restraint mechanism 1300, which comprises the collar 1310, minimizes the risk of accidental needle sticks by preventing the Huber needle 110 from being pulled up and out of the base 300'.

It is to be understood that the Huber needle assembly 1200 may be used in the steps S1-S4 for inserting and withdrawing the Huber needle assembly 1200 from the subcutaneous infusion port 800. Use of the Huber needle assembly 1200 is similar to that of the Huber needle assembly 100. A notable difference is how the tip block and safety or restraint mechanisms are implemented.

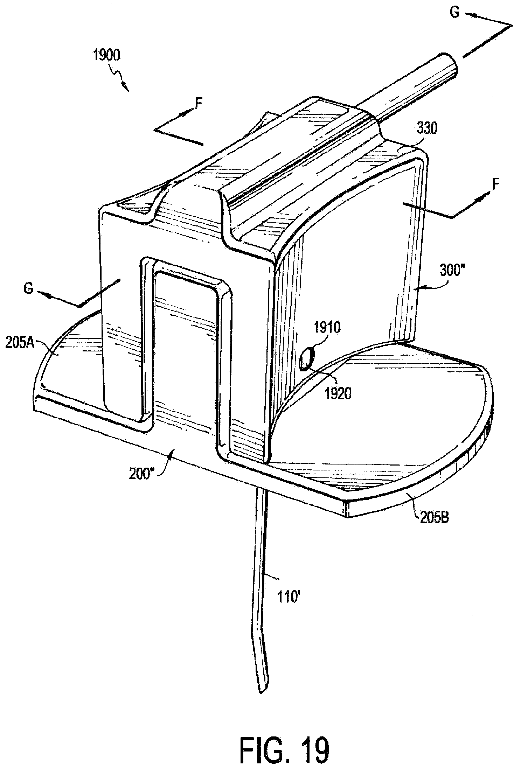

Illustrated in FIG. 19 is yet another alternative exemplary embodiment of the Huber needle assembly 100 or 1200, generally designated as 1900 in the figure, in accordance with an exemplary embodiment of the present invention. The Huber needle assembly 1900 includes numerous elements in common with the Huber needle assemblies 110 and 1200. Like elements are labeled with the same numerals. It is to be understood that not all of such like elements are identical in form, shape, or function. Several of such elements of the Huber needle assembly 1900, though having similar forms, shapes, or functions to elements of the Huber needle assembly 100 or 1200 having the same numerals, do not have identical forms, shapes, or functions. Several differences in such forms, shapes, or functions are described below. It is to be understood that the description below of such differences is not to be construed as a discussion of all differences between elements of the Huber needle assembly 1900 and those of Huber needle assemblies 100 and 1200. Instead, differences may be apparent from the figures and are not addressed below.

The Huber needle assembly 1900 comprises a Huber needle 110', a base 200", and a housing 300" for the Huber needle 110'. The Huber needle 110' is securely mounted within the housing 300" and is slidably disposed through the base 200". As described in further detail below, the Huber needle 110' is similar to the Huber needle 110, though not identical, as it lacks the first portion 115 and the bend 116. The components of the Huber needle assembly 1900 are formed from plastic. The Huber needle 110 is formed from metal, such as stainless steel.

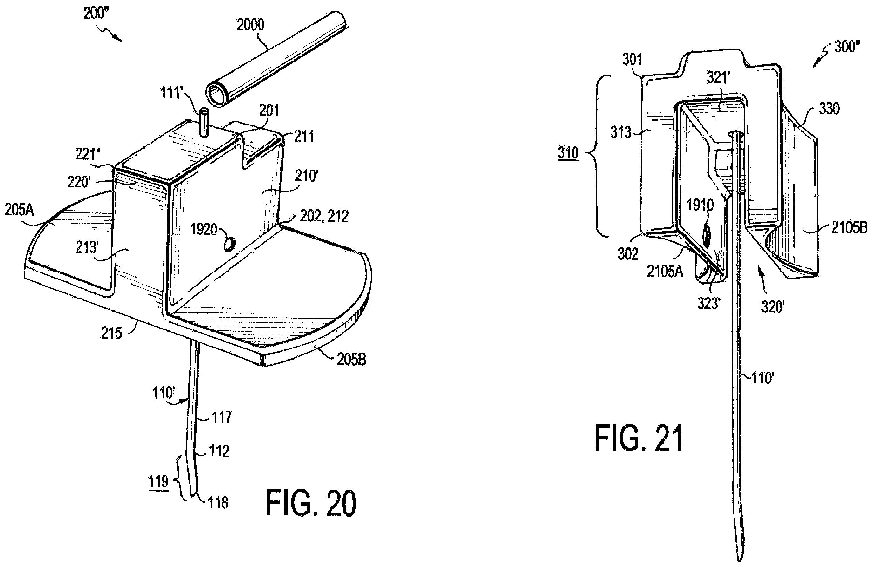

Illustrated in FIG. 20 is a perspective view of the base 200" and the Huber needle 110' slidably disposed therethrough, in accordance with an exemplary embodiment of the present invention. In FIG. 20, the housing 300" of the Huber needle assembly 1200 has been removed to more clearly show the base 200", the Huber needle 110', and the tubing 2000. The base 200" has a first side or end 201 and a second side or end 202. The base 200" comprises the base plate 215 disposed at the second side 202 of the base 200", the pair of wings 205A and 205B connected to one another by the base plate 215, and a body 210' comprising an outer wall 213'. The base 200" has numerous similarities with the base 200 or 200'. For example, the body 210' extends upwardly from the base plate 215; the Huber needle 110' is slidably disposed through the body 210'; etc.

The base 200" differs from the bases 200 and 200' in a few ways. One difference is that body 210' is not cylindrically shaped, as the body 210 is, but instead has the general shape of a rectangular prism. Another difference of the base 200" is a safety or restraint mechanism 2300 that performs the functions of the safety or restraint mechanism 500 of the Huber needle assembly 100, without use of the cable 510, the first stop 513, the second stop 514, or the holes 225 and 325 present in the Huber needle assembly 100, and the functions of the safety or restraint mechanism 1300 without the separate collar 1310. The safety or restraint mechanism 2300 is similar to the safety or restraint mechanism 1300 in that it includes a narrow passage 2310 that extends through the base 200" and that is sized to prevent passage of the bent portion 119 of tip 118 of the Huber needle 110' therethrough. Thus, the Huber needle 110' is prevented from being separated from the base 200". In an exemplary embodiment, the safety or restraint mechanism 2300 is formed from the same material from which the base 200" is formed.

Comparing FIG. 20 to FIGS. 2 and 13A, other differences are observable. For example, the wings 205A and 205B of the base 200" have a different shape from the wings 205A and 205B of the base 200 and the base 200'. Further, the base 200" includes a protrusion 220' that is shaped differently from the protrusion 220 present on the base 200. The protrusion 220' has a general shape of a rectangular prism and comprises a top surface 221".

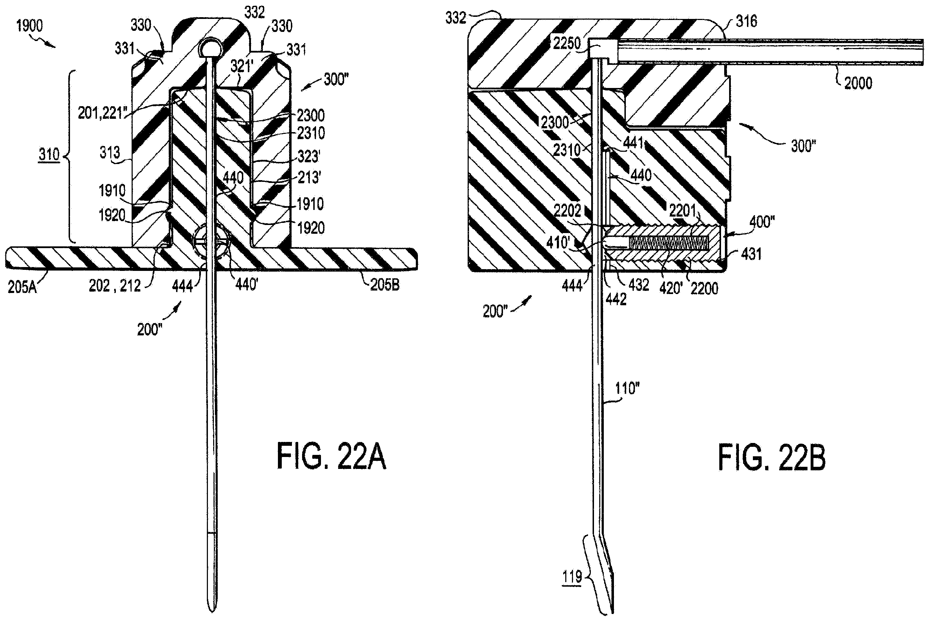

Illustrated in FIG. 21 is a perspective view of the housing or hub 300" for the Huber needle 110' from a bottom of the housing 300", in accordance with an exemplary embodiment of the present invention. The housing 300" comprises the handle 330, which is shaped differently from the handle 330 of the housing 300 or the handle 330 of the housing 300'. The handle 330 includes the top surface 332 (shown in FIGS. 22A and 22B) upon which the nurse or medical practitioner may place his or her hand or digit when using the Huber needle assembly 1900. The housing 300" further includes an interior space 320' comprising an interior wall 323' and an upper surface 321'. The interior space 320' serves the same purpose as the interior space 320 of the Huber needle assemblies 100 and 1200: It receives the base 200", specifically the body 210' and the protrusion 220'. The outer wall 313 of the body 310' of the housing 300" has a general rectangular prism shape, albeit with concave sides 2105A and 2105B that facilitate gripping the Huber needle assembly 1900.

FIG. 22A illustrates a cross-sectional view of the Huber needle assembly 1900 taken along a line F-F illustrated in FIG. 19, in accordance with an exemplary embodiment of the present invention. FIG. 22B illustrates a cross-sectional view of the Huber needle assembly 1900 taken along a line G-G illustrated in FIG. 19, in accordance with an exemplary embodiment of the present invention. FIGS. 22A and 22B illustrate how the base 200" mates with the housing 300". FIGS. 22A and 22B are now described together with reference to FIG. 20.

As illustrated in FIGS. 22A and 22B, the body 210' and protrusion 220' of the base 200" each has a generally rectangular prism shape for fitting within the interior space 320' of the housing 300". When so situated, the top surface 221" of the protrusion 220' is adjacent to the upper surface 321' of the interior space 320'. Additionally, the interior wall 323' of the interior space 320' is adjacent to the outer wall 213' of the base 200". It is to be understood that other shapes and configurations for the bodies 210' and 310' of the base 200" and housing 300", respectively, are contemplated and that they are not limited to the shapes illustrated in the figures.

The Huber needle 110' is securely mounted within the housing 300'. The Huber needle 110' enters the housing 300" through the upper surface 321'. The first end 111' of the Huber needle 110 terminates in a chamber or cavity 2250. The chamber 2250 communicates with a tube 2000 that exits the housing 300" at an end 316. Fluid is administered and withdrawn from the Huber needle 110' via the tube 2000. In an alternative exemplary embodiment of the housing 300", the Huber needle 110 may be used in place of the Huber needle 110'. In such an embodiment, the first portion 115 at the first end 111 of the Huber needle 110 extends into the tube 2000, and the chamber 2250 need not be included in the alternative exemplary embodiment of the housing 300".

In the exemplary embodiment illustrated in FIGS. 22A and 22B, there a gap between the outer wall 213' and the inner wall 323'. Thus, there is a loose fit between the base 200" and the housing 300". Disposed within the outer wall 313 of the body 310' is a pair of dents or openings 1910. These correspond to and receive respective detents 1920 located on the outer wall 213' of the base 200", as best seen in FIGS. 19 and 22A, to maintain the gaps and relative positions of the base 200" and housing 300" during use, e.g., insertion. The detents 1920 resist but do not prevent separation.

In an alternative exemplary embodiment, no such dents or gaps 1910 or detents 1920 exist such that when the base 200" is mated to the housing 300", the outer wall 213' is in contact with the inner wall 323'. In such exemplary embodiment, the base 200" may fit tightly to the housing 300" but be separable from the housing 300" during use.

The Huber needle assembly 1900 includes a needle capture mechanism comprising a tip block mechanism 400" and a safety or restraint mechanism 2300. An exemplary embodiment of the tip block mechanism 400" is illustrated in FIGS. 22A and 22B. Generally, the tip block mechanism 400" is similar to the tip block mechanism 400. A primary difference between the two is that the ball 410 and spring 420 in the Huber needle assembly 100 is replaced by a plunger 410' and a spring 420' within a detent housing 2200. Thus, the tip block mechanism 400" is a detent.

In the base 200", the horizontal ball race 430 comprises a first end 431" and the second end 432. The first end 431" differs from the first end 431 in that it is not a dead end so that the detent housing 2200 may be installed in the base 200" during manufacture. The second end 432 opens to the second end 442 of the vertical needle chamber 440.

The detent housing 2200 is generally cylindrically shaped and includes an internal cavity 2201 in which the plunger 410' and spring 420' are disposed. The spring 420' is disposed at a first end 2201 of the housing 2200 and the plunger 410' is disposed at a second end 2202 thereof. The spring 420' urges the plunger 410' toward the vertical needle chamber 440 and the Huber needle 110'. When the Huber needle 110' is fully disposed through the vertical needle chamber 440, the spring 420' presses the plunger 410' against the Huber needle 110'. The Huber needle 110' blocks the plunger 410' from fully entering the vertical needle chamber 440.

When the Huber needle assembly 1900 is in its pre-insertion or insertion state in which the base 200" is adjacent to the housing 300", the Huber needle 110' is slidably disposed through a passageway 2310 in the base 200". When the Huber needle assembly 1900 is in its withdrawal or post-insertion state in which the base 200" is separated from the housing 300", the bend 119 of the Huber needle 110' is prevented from passing through the passageway 2310 in the base 200". In an exemplary embodiment, the passageway 2310 of the base 200" is sized so that the bend 119 of the Huber needle 110' binds up within the passageway 2310, rather than merely being prevented from entering the passageway 2310.

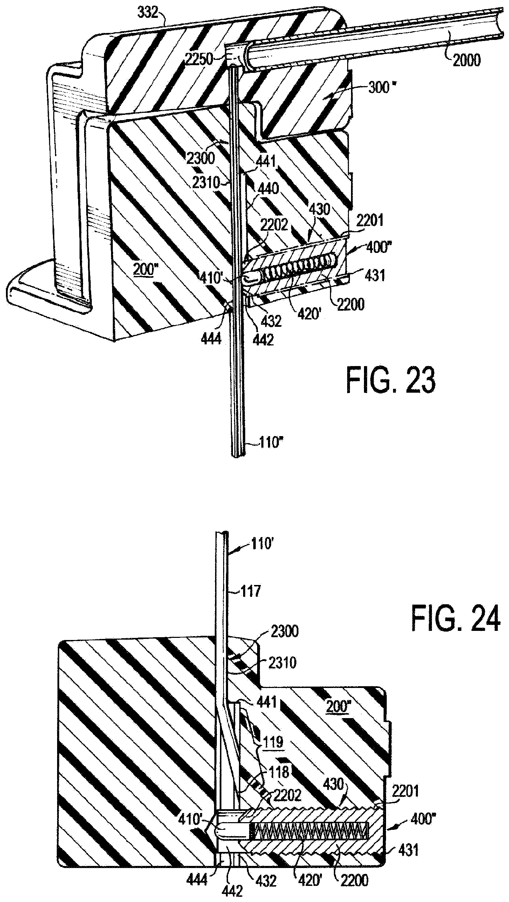

The positions of the components of the tip block mechanism 400" and the safety or restraint mechanism 2300 in the pre-insertion and post-insertion state of the Huber needle assembly 1900 are now described with respect to FIGS. 23 through 25. FIG. 23 illustrates a perspective view of the Huber needle assembly 1900 from the cross-section G-G of FIG. 19, in accordance with an exemplary embodiment of the present invention. Specifically, FIG. 23 illustrates the Huber needle assembly 1900 in its pre-insertion or insertion state in which the base 200" is adjacent to the housing 300". In this state, the plunger 410' is in place within the horizontal ball race 430 at the second end 432 thereof. The plunger 410' is in contact with the second portion 117 (labeled in FIG. 20) of the Huber needle 110'. The spring 420' provides a horizontal force to press the plunger 410' against the Huber needle 110'.

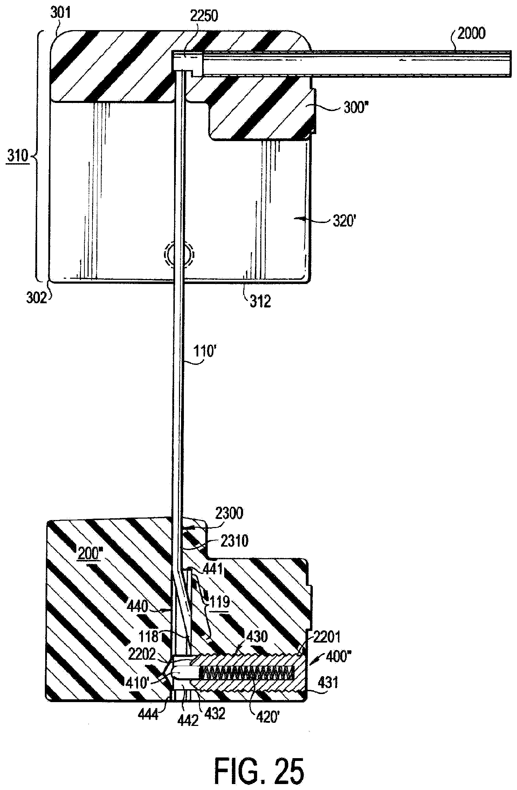

FIGS. 24 and 25 illustrate a perspective view of the base 200" and the Huber needle assembly 1900, respectively, from the cross-section G-G of FIG. 19, in accordance with an exemplary embodiment of the present invention. FIG. 24 illustrates the base 200" of the Huber needle assembly 1900 in its post-insertion position in which the housing 300" has been pulled away from the base 200", and FIG. 25 illustrates the Huber needle assembly 1900 in its post-insertion state in which the housing 300" has been pulled away from the base 200".

As seen in FIGS. 24 and 25, the tip 118 of the Huber needle 110' resides within the vertical needle chamber 440 when the housing 300" has been fully pulled away from the base 200". In such position, the spring 420' has pressed the plunger 410' into the vertical needle chamber 440, thereby blocking the orifice 444 at the second end 442 of the vertical needle chamber 440. The tip 118 of the Huber needle 110 is thereby blocked from passing through the orifice 444. In this way, the tip block mechanism 400" minimizes the risk of accidental needle sticks by blocking the tip 118 from exiting the orifice 444.

When the housing 300" has been pulled away from the base 200" so that the tip 118 of the Huber needle 110' is restrained within the vertical needle chamber 440, the bend 119 of the Huber needle 110 comes into contact with the passageway 2310. The bend 119 of the Huber needle 110' is prevented from passing through the passageway 2310 because it is larger than the diameter of the passageway 2310. Further lateral movement of the base 200" away from the housing 300" is prevented by the fact that the bend 119 cannot pass through the passageway 2310. In this way, the safety or restraint mechanism 2300, which comprises the passageway 2310, minimizes the risk of accidental needle sticks by preventing the Huber needle 110' from being pulled up and out of the base 300".

It is to be understood that the Huber needle assembly 1900 may be used in the steps S1-S4 for inserting and withdrawing the Huber needle assembly 1900 from the subcutaneous infusion port 800. Use of the Huber needle assembly 1900 is similar to that of the Huber needle assemblies 100 and 1200. A notable difference is how the tip block and safety or restraint mechanisms are implemented.

In yet another exemplary embodiment of the Huber needle assembly of the present invention, there is included in the outer wall of the housing of the Huber needle assembly a plurality of holes, and there is included in the outer wall of the base a plurality of corresponding detents. The detents may selectively engage respective ones of the holes to position the base at a desired distance from the housing so that the length of the Huber needle extending from the base may be set at a desired amount.

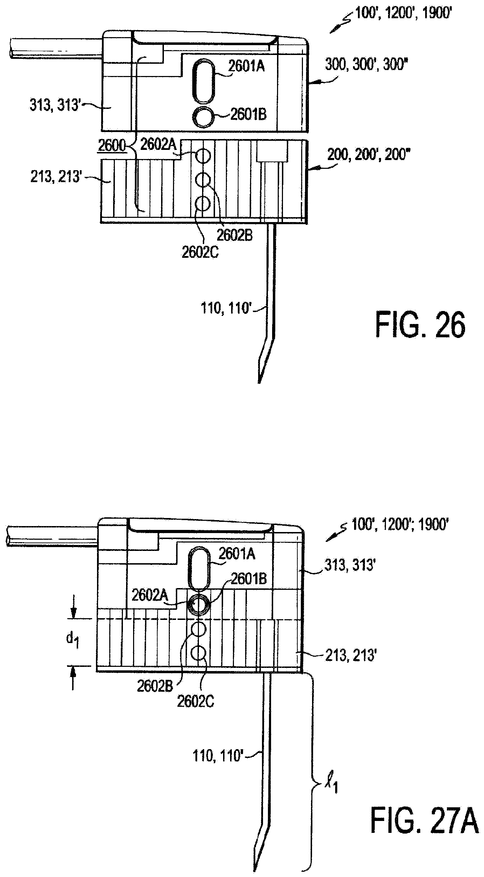

Referring now to FIG. 26, there are illustrated exemplary alternative embodiments of the Huber needle assemblies 100, 1200, and 1900, respectively designated as 100', 1200', and 1900', in accordance with an exemplary embodiment of the present invention. In the exemplary embodiments illustrated the outer wall 313 or 313' of the housings 300, 300', or 300" of the Huber needle assemblies 100', 1200', and 1900' comprises a plurality of holes 2601A and 2601B, and the outer wall 213 or 213' of the bases 200, 200', or 200" of the Huber needle assemblies 100', 1200', and 1900' comprises a plurality of detents 2602A, 2602B, and 2602C. The hole 2601B is positioned to selectively engage one of the detents 2602A, 2602B, and 2602C. The plurality of holes 2601A and 2601B and plurality of detents 2602A, 2602B, and 2602C form a Huber needle 110, 110' length selection means 2600.

When the hole 2601B is positioned to selectively engage the detent 2602A, the housing 300, 300', 300" is separated from the base 200, 200', 200" by a first distance d₁, as illustrated in FIG. 27A. The tip 118 of the Huber needle 110, 110' extends from the bottom of the base 200, 200', 200" by a length l₁.

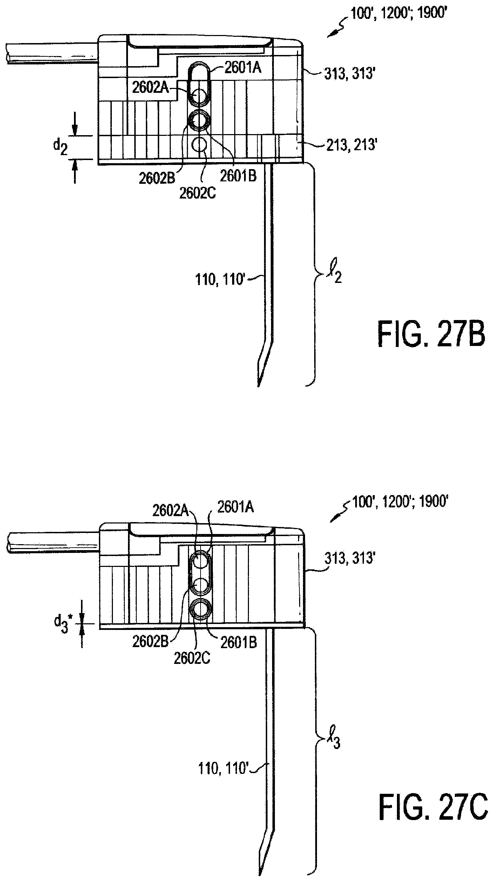

When the hole 2601B is positioned to selectively engage the detent 2602B, the housing 300, 300', 300" is separated from the base 200, 200', 200" by a second distance d₂, which is less than d₁, as illustrated in FIG. 27B. The tip 118 of the Huber needle 110, 110' extends from the bottom of the base 200, 200', 200" by a length l₂, which is greater than l₁. The detent 2602A is disposed within the hole 2601A.

When the hole 2601B is positioned to selectively engage the detent 2602C, the housing 300, 300', 300" is separated from the base 200, 200', 200" by a second distance d₃, which is less than d₂ and about 0 inches (0 mm), as illustrated in FIG. 27C. The tip 118 of the Huber needle 110, 110' extends from the bottom of the base 200, 200', 200" by a length l₃, which is greater than l₂. The detents 2602A and 2602B are disposed within the hole 2601A.

It is to be understood that the Huber needle assembly 100', 1200', 1900' may be used in the steps S1-S4 for inserting and withdrawing the Huber needle assembly 100', 1200', 1900' from the subcutaneous infusion port 800. The Huber needle 110, 110' length selection means 2600 allows a practitioner to select the length of the Huber needle 110, 110' extending from the base 200, 200', 200" depending on a thickness of a patient's skin and tissue 850 when using the Huber needle assembly 100', 1200', 1900' during the steps S1-S4. By setting this length, the practitioner may ensure that the wings 205A and 205B come to rest against the skin 850 when the Huber needle 110, 110' is fully inserted into the port 800. Therefore, the practitioner can ensure that the Huber needle assembly 100', 1200', 1900' can be securely taped to the patient's skin 850.

It is to be understood that the combinations of elements of the embodiments of the Huber needle assemblies 100, 1200, and 1900 and exemplary alternative embodiments thereof are exemplary. Other combinations of the illustrated and described elements are contemplated. For example, an alternative exemplary embodiment in which a vertical ball race 430' is used in the base 200 or 200" is contemplated. Further, the base 200' or 200" may be used in the Huber needle assembly 100 to provide a redundant restraint mechanism. Alternatively, the horizontal ball race 430 may be used in the Huber needle assembly 1200, or the tip block mechanism 400" may be used in the Huber needle assemblies 100 or 1200. Thus, it is to be understood, that any components of the Huber needle assemblies 100, 1200, or 1900 may be used to form a Huber needle assembly having a combination of components not illustrated in the figures. Further, it is contemplated that any of these alternative embodiments may be used in the steps S1-S4 for inserting and withdrawing the Huber needles of such embodiments from the subcutaneous infusion port 800.

As used herein, the tip block mechanism 400 and the tip block mechanism 400' or 400" is a needle tip block means for blocking the tip 118 of the Huber needle assembly 100, 1200, or 1900, respectively. In its most basic configuration, the tip block means comprises the ball 410 and spring 420 or the detent 400". It is to be understood that the tip block means may also further comprise the cup 445 and/or the ramp 433 (in the configuration for the tip block mechanism 400').

As also used herein, the safety or restraint mechanism 500, 1300, or 2300 is a restraint means for restraining movement of the housing 300 (or 300' or 300") relative to the base 200 (or 200' or 200"). Such restraint means may include the cable 510, the hole 1312, or the passageway 2310. In its most basic configuration, the restraint means includes a slidable anchor 513 disposed within the housing 300 (or 300' or 300") and an anchor 514 disposed within the base 200 (or 200' or 200"). The anchor 514 may be fixed in the base 200 (or 200' or 200") or slidable. The restraint means may alternatively or additionally include the collar 1310 with or without the protrusion 1320.

These and other advantages of the present invention will be apparent to those skilled in the art from the foregoing specification. Accordingly, it is to be recognized by those skilled in the art that changes or modifications may be made to the above-described embodiments without departing from the broad inventive concepts of the invention. It is to be understood that this invention is not limited to the particular embodiments described herein, but is intended to include all changes and modifications that are within the scope of the invention.

## Claims

1. A Huber needle assembly (1900) comprising:
a housing (300");
a Huber needle (110', 110") securely mounted within the housing (300), the Huber needle (110', 110") comprising a tip (118) and a bent portion (119);
a spring (420'); and
a base (200', 200") through which the Huber needle (110) is slidably disposed so that when the housing (300', 300") is moved away from the base (200', 200") the Huber needle (110) traverses the base (200', 200") to draw the tip (118) within the base (200', 200"), the base comprising a needle tip blocker (410') slidably disposed within the base (200', 200") to block the tip (118) of the Huber needle (110) when the tip (118) of the Huber needle (110) is retracted into the base (200', 200");
wherein the base (200', 200") further comprises:
a vertical chamber (440) through which the needle (110) is slidably disposed;
a chamber (430') in which the needle tip blocker (410') and the spring (420') are slidably disposed;
wherein the base (200', 200") comprises a protrusion (220, 220') and the housing (300', 300") comprises an interior space (320, 320'), the protrusion (220, 220') configured to be disposed within the interior space (320, 320') when the Huber needle assembly (1200, 1900) is in an insertion position,
wherein:
the vertical chamber (440) has a first end (441) and a second end (442),
**characterized in that,**
the tip block mechanism (400") is a detent, wherein the tip blocker is a plunger (410') and the spring (420') is within a detent housing (2200),
wherein the detent housing (2200) is generally cylindrically shaped and includes an internal cavity (2201) in which the plunger (410') and spring (420') are disposed,
wherein when the housing (300") has been fully pulled away from the base (200"), the spring (420') presses the plunger (410') into the vertical needle chamber (440), thereby blocking the orifice (444) at the second end (442) of the vertical needle chamber (440).

2. The Huber needle assembly (1900) of claim 1, wherein the chamber (430) in which the needle tip blocker (410') is slidably disposed is a horizontal chamber (430).

3. The Huber needle assembly (1900) of claim 1 or 2, wherein:
the base (220, 220') comprises a top surface (221, 221'), a bottom surface (1332), a chamber (440) comprising a first orifice (443) through the top surface to the chamber (440), and a second orifice (444) through the bottom surface (1332) to the chamber (444), and
the Huber needle (110) is slidably disposed through the first orifice (443), the chamber (440), and the second orifice (444).

4. The Huber needle assembly (1900) according to any of the preceding claims, wherein the housing (300', 300") includes a handle (330) formed on a first end, the handle (330) connected to the housing (300', 300") by a contoured surface (314).

5. The Huber needle assembly (1900) of claim 4, wherein the handle (330) includes a flare (331) for assisting the use of the Huber needle assembly (1900) to insert and withdraw the tip (118) of the Huber needle (110) from a subcutaneously implanted venous access port.

## Patentansprüche

1. Eine Huber-Nadelanordnung (1900) bestehend aus:
einem Gehäuse (300");
eine Huber-Nadel (110', 110"), die gesichert in dem Gehäuse (300) montiert ist, wobei die Huber-Nadel (110', 110") eine Spitze (118) und einen gebogenen Abschnitt (119) aufweist;
eine Feder (420'); und
eine Basis (200', 200"), durch die die Huber-Nadel (110) verschiebbar angeordnet ist, so dass, wenn das Gehäuse (300', 300") von der Basis (200', 200") wegbewegt wird, die Huber-Nadel (110) die Basis (200', 200") durchquert, um die Spitze (118) innerhalb der Basis (200', 200") zu ziehen, wobei die Basis einen Nadelspitzenblockierer (410') umfasst, der gleitend in der Basis (200', 200") angeordnet ist, um die Spitze (118) der Huber-Nadel (110) zu blockieren, wenn die Spitze (118) der Huber-Nadel (110) in die Basis (200', 200") zurückgezogen wird;
wobei die Basis (200', 200") ferner umfasst:
eine vertikale Kammer (440), durch die die Nadel (110) verschiebbar angeordnet ist;
eine Kammer (430'), in der der Nadelspitzenblockierer (410') und die Feder (420') verschiebbar angeordnet sind;
wobei die Basis (200', 200") einen Vorsprung (220, 220') und das Gehäuse (300', 300") einen Innenraum (320, 320') umfasst, wobei der Vorsprung (220, 220') so konfiguriert ist, dass er innerhalb des Innenraums (320, 320') angeordnet ist, wenn sich die Huber-Nadelanordnung (1200, 1900) in einer Einführposition befindet,
wobei:
die vertikale Kammer (440) ein erstes Ende (441) und ein zweites Ende (442) hat,
**dadurch gekennzeichnet**,
der Spitzenblockierungsmechanismus (400") eine Raste ist, wobei der Spitzenblockierer ein Kolben (410') ist und die Feder (420') sich innerhalb eines Rastgehäuses (2200) befindet,
wobei das Rastgehäuse (2200) allgemein zylindrisch geformt ist und einen inneren Hohlraum (2201) aufweist, in dem der Kolben (410') und die Feder (420') angeordnet sind,
wobei die Feder (420') den Kolben (410') in die vertikale Nadelkammer (440) drückt, wenn das Gehäuse (300") vollständig von der Basis (200") weggezogen worden ist, und dadurch die Öffnung (444) am zweiten Ende (442) der vertikalen Nadelkammer (440) blockiert.

2. Die Huber-Nadelanordnung (1900) nach Anspruch 1, wobei die Kammer (430), in der der Nadelspitzenblockierer (410') verschiebbar angeordnet ist, eine horizontale Kammer (430) ist.

3. Die Huber-Nadelanordnung (1900) nach Anspruch 1 oder 2, wobei:
die Basis (220, 220') eine obere Fläche (221, 221'), eine untere Fläche (1332), eine Kammer (440) aufweisend eine erste Öffnung (443) durch die obere Fläche zur Kammer (440) und eine zweite Öffnung (444) durch die untere Fläche (1332) zur Kammer (444) umfasst, und die Huber-Nadel (110) durch die erste Öffnung (443), die Kammer (440) und die zweite Öffnung (444) verschiebbar angeordnet ist.

4. Die Huber-Nadelanordnung (1900) nach einem der vorstehenden Ansprüche, wobei das Gehäuse (300', 300") einen an einem ersten Ende ausgebildeten Griff (330) aufweist, der mit dem Gehäuse (300', 300") durch eine konturierte Oberfläche (314) verbunden ist.

5. Die Huber-Nadelanordnung (1900) nach Anspruch 4, wobei der Griff (330) eine Aufweitung (331) zur Unterstützung der Verwendung der Huber-Nadelanordnung (1900) zum Einführen und Zurückziehen der Spitze (118) der Huber-Nadel (110) aus einem subkutan implantierten venösen Zugangsport aufweist.

## Revendications

1. Un ensemble d'aiguilles de Huber (1900) comprenant :
un boîtier (300") ;
une aiguille de Huber (110', 110") solidement montée dans le logement (300), l'aiguille de Huber (110', 110") comprenant une pointe (118) et une partie courbée (119) ;
un ressort (420') ; et
une base (200', 200") à travers laquelle l'aiguille de Huber (110) est disposée de façon coulissante de sorte que lorsque le boîtier (300', 300") est éloigné de la base (200', 200"), l'aiguille de Huber (110) traverse la base (200', 200") pour tirer la pointe (118) à l'intérieur de la base (200', 200"), la base comprenant un bloqueur de pointe d'aiguille (410') disposé de manière coulissante dans la base (200', 200") pour bloquer la pointe (118) de l'aiguille de Huber (110) lorsque la pointe (118) de l'aiguille de Huber (110) est rétractée dans la base (200', 200") ;
dans lequel la base (200', 200") comprend en outre :
une chambre verticale (440) à travers laquelle l'aiguille (110) est disposée de manière coulissante ;
une chambre (430') dans laquelle le bloqueur de pointe d'aiguille (410') et le ressort (420') sont disposés de manière coulissante ;
dans laquelle la base (200', 200") comprend une saillie (220, 220') et le logement (300', 300") comprend un espace intérieur (320, 320'), la saillie (220, 220') étant configurée pour être disposée dans l'espace intérieur (320, 320') lorsque l'ensemble d'aiguille de Huber (1200, 1900) est en position d'insertion,
dans lequel:
la chambre verticale (440) a une première extrémité (441) et une deuxième extrémité (442),
**caractérisé en ce que**,
le mécanisme de blocage de la pointe (400")
est un cran d'arrêt, dans lequel le bloqueur de pointe est un piston (410') et le ressort (420') se trouve dans un boîtier de cran d'arrêt (2200),
dans lequel le boîtier de cran d'arrêt (2200) est généralement de forme cylindrique et comprend une cavité interne (2201) dans laquelle sont disposés le plongeur (410') et le ressort (420'),
dans lequel, lorsque le boîtier (300") a été complètement retiré de la base (200"), le ressort (420') presse le plongeur (410') dans la chambre à aiguille verticale (440), bloquant ainsi l'orifice (444) à la deuxième extrémité (442) de la chambre à aiguille verticale (440).

2. L'ensemble d'aiguilles de Huber (1900) de la revendication 1, dans lequel la chambre (430) dans laquelle le bloqueur de pointe d'aiguille (410') est disposé de manière coulissante est une chambre horizontale (430).

3. L'ensemble d'aiguilles de Huber (1900) selon la revendication 1 ou 2, dans lequel :
la base (220, 220') comprend une surface supérieure (221, 221'), une surface inférieure (1332), une chambre (440) comprenant un premier orifice (443) à travers la surface supérieure vers la chambre (440), et un deuxième orifice (444) à travers la surface inférieure (1332) vers la chambre (444), et l'aiguille de Huber (110) est disposée de manière coulissante à travers le premier orifice (443), la chambre (440) et le deuxième orifice (444).

4. L'ensemble aiguille de Huber (1900) selon l'une des revendications précédentes, dans lequel le boîtier (300', 300") comprend une poignée (330) formée sur une première extrémité, la poignée (330) étant reliée au boîtier (300', 300") par une surface profilée (314).

5. L'ensemble d'aiguilles de Huber (1900) selon la revendication 4, dans lequel la poignée (330) comprend un évasement (331) pour aider à l'utilisation de l'ensemble d'aiguille de Huber (1900) pour insérer et retirer la pointe (118) de l'aiguille de Huber (110) d'un orifice d'accès veineux implanté de manière sous-cutanée.
